# EUROPEAN PATENT APPLICATION

(11) **EP 4 656 139 A2**
(43) Date of publication of application: **03.12.2025**
(21) Application number: 25203346.9
(22) Date of filing: 29.03.2024
(51) Int. Cl.: A61B 8/00

(54) **GRAPHICAL USER INTERFACE FOR INTRAVASCULAR PLAQUE BURDEN INDICATION**

(30) Priority: 30.03.2023 US 202363455860 P
(62) Divisional of application: 24722384.5
(71) Applicant: Boston Scientific Scimed, Inc., Maple Grove, MN 55311-1566 (US)
(72) Inventor: The designation of the inventor has not yet been filed
(74) Representative: Vossius & Partner Patentanwälte Rechtsanwälte mbB

(57) **Abstract**

The present invention relates to an apparatus, comprising a processor coupled to a memory, the memory comprising instructions executable by the processor, the processor configured to couple to an intravascular ultrasound, IVUS, imaging system and configured to execute the instructions, which instructions when executed cause the apparatus to receive a series of intravascular ultrasound, IVUS, images of a vessel of a patient, the series of IVUS images comprising a plurality of frames, generate a GUI comprising a longitudinal view of the vessel, and render the GUI for display on a display.

## Description

### CROSS-REFERENCE TO RELATED APPLICATIONS

This application claims the benefit of U.S. Provisional Patent Application Serial No. 63/455,860 filed on March 30, 2023, the disclosure of which is incorporated herein by reference.

### TECHNICAL FIELD

The present disclosure generally relates to intravascular ultrasound (IVUS) imaging systems. Particularly, but not exclusively, the present disclosure relates to an improved graphical user interface for IVUS imaging systems.

### BACKGROUND

Ultrasound devices insertable into patients have proven diagnostic capabilities for a variety of diseases and disorders. For example, intravascular ultrasound (IVUS) imaging systems have been used as an imaging modality for diagnosing blocked blood vessels and providing information to aid medical practitioners in selecting and placing stents and other devices to restore or increase blood flow.

IVUS imaging systems include a control module (with a pulse generator, an image acquisition and processing components, and a monitor), a catheter, and a transducer disposed in the catheter. The transducer-containing catheter is positioned in a lumen or cavity within, or in proximity to, a region to be imaged, such as a blood vessel wall or patient tissue in proximity to a blood vessel wall. The pulse generator in the control module generates electrical pulses that are delivered to the transducer and transformed to acoustic pulses that are transmitted through patient tissue. The patient tissue (or other structure) reflects the acoustic pulses and reflected pulses are absorbed by the transducer and transformed to electric pulses. The transformed electric pulses are delivered to the image acquisition and processing components and converted into images displayable on the monitor.

IVUS systems can be used to image a vessel to determine the existence of an intravascular obstruction or stenosis, as well as to determine the nature and degree of the obstruction or stenosis. Further, IVUS systems can be used to visualize segments of a vascular system that may be difficult to visualize using other intravascular imaging techniques, such as angiography, due to, for example, movement (e.g., a beating heart) or obstruction by one or more structures (e.g., one or more blood vessels not desired to be imaged). Thus, there is a need for user interfaces, and particularly graphical user interfaces, that communicate information from the IVUS system to a user related to the nature and degree of obstruction or stenosis of a vessel.

### BRIEF SUMMARY

This Summary is provided to introduce a selection of concepts in a simplified form that are further described below in the Detailed Description. This Summary is not intended to necessarily identify key features or essential features of the claimed subject matter, nor is it intended as an aid in determining the scope of the claimed subject matter.

In general, the present disclosure provides an improvement to computing devices and particularly to IVUS guidance systems in that the present disclosure provides a graphical user interface arranged to convey the wealth of information with which modern IVUS systems generate. For example, an IVUS system may include machine learning features to process and analyze the signals generated during an IVUS run. Such information can include automatic detection of obstructions or stenosis (e.g., plaque burden) within a vessel. The improved graphical user interface provided herein includes displaying such information as well as providing a method for a user to manipulate the information as needed.

It is important to note that the present disclosure can be provided to assist physicians in identifying regions of a vessel where the plaque burden is less than a threshold level (e.g., 50%, or the like). Such information can be useful to identify landing zones for a stent (e.g., prior to a percutaneous coronary intervention (PCI) treatment) as well as to identify a misplaced stent (e.g., after a PCI treatment).

With some embodiments, the disclosure can be implemented as a method. The method can comprise receiving a series of intravascular ultrasound (IVUS) images of a vessel of a patient, the series of IVUS images comprising a plurality of frames; receiving, for each of the plurality of frames, an indication of plaque burden in the vessel; generating a graphical component comprising an indication of the plaque burden respective to a threshold amount; generating a GUI comprising a longitudinal view of the vessel and the graphical component configured to indicate the detected plaque burden along a longitudinal axis of the vessel; and rendering the GUI for display on a display.

In further embodiments of the method, the graphical component is a first graphical component, and the method comprises: generating a second graphical component comprising the longitudinal view of the vessel; and generating the GUI comprising the first graphical component and the second graphical component.

In further embodiments, the method comprises generating the GUI comprising overlaying the first graphical component over a portion of the second graphical component to indicate the plaque burden along the longitudinal axis of the vessel.

In further embodiments of the method, the first graphical component comprises a line disposed along a portion of the longitudinal axis of the vessel.

In further embodiments, the method comprises: identifying, for each of the plurality of frames, whether the plaque burden is less than a threshold level; and overlaying the line over portions of the second graphical component associated with the ones of the plurality of frames where the plaque burden is less than the threshold level.

In further embodiments of the method, the second graphical component comprises an indication of a distal bracket, a proximal bracket, a minimum region, a vessel profile view, and a mirror of the vessel profile view.

In further embodiments of the method, the vessel profile view comprises a graphical representation of a vessel border and a lumen border of the vessel along the longitudinal axis.

In further embodiments of the method, the distal bracket comprises one of a plurality of bracket graphical representations selected based on the location of the distal bracket relative to the plaque burden and the threshold level.

In further embodiments, the method comprises: identifying a location of the distal bracket; determining whether the distal bracket is associated with a frame of the plurality of frames where the plaque burden is less than the threshold level; and selecting a first one of the plurality of bracket graphical representations based on a determination that the distal bracket is associated with a frame of the plurality of frames where the plaque burden is less than the threshold level; or selecting a second one of the plurality of bracket graphical representations based on a determination that the distal bracket is not associated with a frame of the plurality of frames where the plaque burden is less than the threshold level.

In further embodiments of the method, the proximal bracket comprises one of a plurality of graphical representations selected based on the location of the proximal bracket relative to the plaque burden and the threshold level.

In further embodiments, the method comprises: identifying a location of the proximal bracket; determining whether the proximal bracket is associated with a frame of the plurality of frames where the plaque burden is less than the threshold level; and selecting a first one of the plurality of bracket graphical representations based on a determination that the proximal bracket is associated with a frame of the plurality of frames where the plaque burden is less than the threshold level; or selecting a second one of the plurality of bracket graphical representations based on a determination that the proximal bracket is not associated with a frame of the plurality of frames where the plaque burden is less than the threshold level.

In further embodiments, the method comprises: generating a third graphical component comprising a cross-section view of a one of the plurality of frames; and generating the GUI comprising the first graphical component, the second graphical component, and the third graphical component.

In further embodiments of the method, the GUI comprises an angiographic image of the vessel.

With some embodiments, the disclosure can be implemented as an apparatus, comprising a processor coupled to a memory, the memory comprising instructions executable by the processor, the processor configured to couple to an intravascular ultrasound (IVUS) imaging system and configured to execute the instructions, which instructions when executed cause the processor to implement the method of any embodiments of the disclosure.

In some embodiments, the disclosure can be implemented as at least one machine readable storage device, comprising a plurality of instructions that in response to being executed by a processor of an intravascular ultrasound (IVUS) imaging system cause the processor to implement the method any embodiments of the disclosure.

With some embodiments, the disclosure can be implemented as a computing apparatus for a medical device. The computing apparatus can comprise: a processor; an interface coupled to the processor, the interface to couple to an intravascular ultrasound (IVUS) device; and a memory coupled to the processor, the memory comprising instructions executable by the processor, which when executed the processor cause the computing apparatus to: receive a series of IVUS images of a vessel of a patient from the IVUS device, the series of IVUS images comprising a plurality of frames, receive, for each of the plurality of frames, an indication of plaque burden in the vessel, generate a graphical component comprising an indication of the plaque burden respective to a threshold amount, generate a GUI comprising a longitudinal view of the vessel and the graphical component configured to indicate the detected plaque burden along a longitudinal axis of the vessel, and render the GUI for display on a display.

In further embodiments of the computing apparatus, the graphical component is a first graphical component, and the instructions, when executed by the processor further cause the computing apparatus to: generate a second graphical component comprising the longitudinal view of the vessel; and generate the GUI comprising the first graphical component and the second graphical component.

In further embodiments of the computing apparatus, the instructions, when executed by the processor further cause the computing apparatus to overlay the first graphical component over a portion of the second graphical component to indicate the plaque burden along the longitudinal axis of the vessel.

In further embodiments of the computing apparatus, the first graphical component comprises a line disposed along a portion of the longitudinal axis of the vessel.

In further embodiments of the computing apparatus, the instructions, when executed by the processor further cause the computing apparatus to identify, for each of the plurality of frames, whether the plaque burden is less than a threshold level; overlay the line over portions of the second graphical component associated with the ones of the plurality of frames where the plaque burden is less than the threshold level.

In further embodiments of the computing apparatus, the second graphical component comprises an indication of a distal bracket, a proximal bracket, a minimum region, a vessel profile view, and a mirror of the vessel profile view and wherein the vessel profile view comprises a graphical representation of a vessel border and a lumen border of the vessel along the longitudinal axis.

In further embodiments of the computing apparatus, the distal bracket comprises one of a plurality of bracket graphical representations selected based on the location of the distal bracket relative to the plaque burden and the threshold level.

In further embodiments of the computing apparatus, the instructions, when executed by the processor further cause the computing apparatus to: identify a location of the distal bracket; determine whether the distal bracket is associated with a frame of the plurality of frames where the plaque burden is less than the threshold level; and select a first one of the plurality of bracket graphical representations based on a determination that the distal bracket is associated with a frame of the plurality of frames where the plaque burden is less than the threshold level; or select a second one of the plurality of bracket graphical representations based on a determination that the distal bracket is not associated with a frame of the plurality of frames where the plaque burden is less than the threshold level.

In further embodiments of the computing apparatus, the proximal bracket comprises one of a plurality of graphical representations selected based on the location of the proximal bracket relative to the plaque burden and the threshold level.

In further embodiments of the computing apparatus, the instructions, when executed by the processor further cause the computing apparatus to: identify a location of the proximal bracket; determine whether the proximal bracket is associated with a frame of the plurality of frames where the plaque burden is less than the threshold level; and select a first one of the plurality of bracket graphical representations based on a determination that the proximal bracket is associated with a frame of the plurality of frames where the plaque burden is less than the threshold level; or selecting a second one of the plurality of bracket graphical representations based on a determination that the proximal bracket is not associated with a frame of the plurality of frames where the plaque burden is less than the threshold level.

In further embodiments of the computing apparatus, the instructions, when executed by the processor further cause the computing apparatus to generate a third graphical component comprising a cross-section view of a one of the plurality of frames; and generate the GUI comprising the first graphical component, the second graphical component, and the third graphical component, wherein the GUI comprises an angiographic image of the vessel.

In further embodiments, the computing apparatus comprises the IVUS imaging device.

With some embodiments, the disclosure can be implemented as at least one machine readable storage device, comprising a plurality of instructions that in response to being executed by a processor of an intravascular ultrasound (IVUS) imaging system comprising an IVUS device, cause the IVUS imaging system to: receive a series of IVUS images of a vessel of a patient from the IVUS device, the series of IVUS images comprising a plurality of frames; receive, for each of the plurality of frames, an indication of plaque burden in the vessel; generate a graphical component comprising an indication of the plaque burden respective to a threshold amount; generate a GUI comprising a longitudinal view of the vessel and the graphical component configured to indicate the detected plaque burden along a longitudinal axis of the vessel; and render the GUI for display on a display.

In further embodiments of the at least one machine readable storage device the graphical component is a first graphical component, and the instructions, when executed by the processor further cause the IVUS imaging system to: generate a second graphical component comprising the longitudinal view of the vessel; generate the GUI comprising the first graphical component and the second graphical component; and overlay the first graphical component over a portion of the second graphical component to indicate the plaque burden along the longitudinal axis of the vessel.

In further embodiments of the at least one machine readable storage device the first graphical component comprises a line disposed along a portion of the longitudinal axis of the vessel, and the instructions, when executed by the processor further cause the IVUS imaging system to: identify, for each of the plurality of frames, whether the plaque burden is less than a threshold level; and overlay the line over portions of the second graphical component associated with the ones of the plurality of frames where the plaque burden is less than the threshold level.

In further embodiments of the at least one machine readable storage device the second graphical component comprises an indication of a distal bracket, a proximal bracket, a minimum region, a vessel profile view, and a mirror of the vessel profile view and wherein the vessel profile view comprises a graphical representation of a vessel border and a lumen border of the vessel along the longitudinal axis.

In further embodiments of the at least one machine readable storage device the distal bracket comprises one of a plurality of bracket graphical representations selected based on the location of the distal bracket relative to the plaque burden and the threshold level, and the instructions, when executed by the processor further cause the IVUS imaging system to: identify a location of the distal bracket; determine whether the distal bracket is associated with a frame of the plurality of frames where the plaque burden is less than the threshold level; and select a first one of the plurality of bracket graphical representations based on a determination that the distal bracket is associated with a frame of the plurality of frames where the plaque burden is less than the threshold level; or select a second one of the plurality of bracket graphical representations based on a determination that the distal bracket is not associated with a frame of the plurality of frames where the plaque burden is less than the threshold level.

### BRIEF DESCRIPTION OF THE SEVERAL VIEWS OF THE DRAWINGS

To easily identify the discussion of any element or act, the most significant digit or digits in a reference number refer to the figure number in which that element is first introduced.
FIG. 1 illustrates an aspect of the subject matter in accordance with one embodiment.
FIG. 2 illustrates an aspect of the subject matter in accordance with one embodiment.
FIG. 3A illustrates an aspect of the subject matter in accordance with one embodiment.
FIG. 3B illustrates an aspect of the subject matter in accordance with one embodiment.
FIG. 4 illustrates IVUS image visualization system 400, in accordance with embodiment(s) of the present disclosure.
FIG. 5A illustrates an aspect of the subject matter in accordance with one embodiment.
FIG. 5B illustrates an aspect of the subject matter in accordance with one embodiment.
FIG. 6 illustrates an aspect of the subject matter in accordance with one embodiment.
FIG. 7 illustrates an aspect of the subject matter in accordance with one embodiment.
FIG. 8 illustrates a logic flow 800 in accordance with one embodiment.
FIG. 9 illustrates a computer-readable storage medium 900 in accordance with one embodiment.
FIG. 10 illustrates a diagrammatic representation of a machine 1000 in the form of a computer system within which a set of instructions may be executed for causing the machine to perform any one or more of the methodologies discussed herein, according to an example embodiment.

### DETAILED DESCRIPTION

The foregoing has broadly outlined the features and technical advantages of the present disclosure such that the following detailed description of the disclosure may be better understood. It is to be appreciated by those skilled in the art that the embodiments disclosed may be readily utilized as a basis for modifying or designing other structures for carrying out the same purposes of the present disclosure. The novel features of the disclosure, both as to its organization and operation, together with further objects and advantages will be better understood from the following description when considered in connection with the accompanying figures. It is to be expressly understood, however, that each of the figures is provided for the purpose of illustration and description and is not intended as a definition of the limits of the present disclosure.

As noted, the present disclosure relates to IVUS systems and automatic assessment of the IVUS images. In particular, the disclosure provides a graphical user interface (GUI) arranged to convey information related to the IVUS images and lesion assessment and provide for the user to manipulate the information. As such, an example IVUS imaging system, patient vessel, and series of IVUS images are described.

Suitable IVUS imaging systems include, but are not limited to, one or more transducers disposed on a distal end of a catheter configured and arranged for percutaneous insertion into a patient. Examples of IVUS imaging systems with catheters are found in, for example, U.S. Pat. Numbers 7,246,959; 7,306,561; and 6,945,938; as well as U.S. Patent Application Publication Numbers 2006/0100522; 2006/0106320; 2006/0173350; 2006/0253028; 2007/0016054; and 2007/0038111; all of which are incorporated herein by reference.

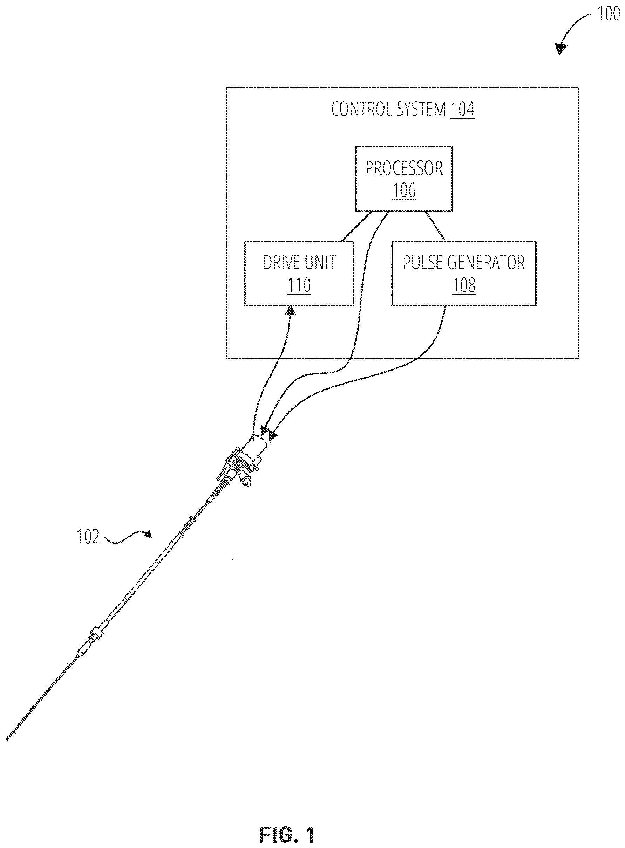

FIG. 1 illustrates one embodiment of an IVUS imaging system 100. The IVUS imaging system 100 includes a catheter 102 that is couplable to a control system 104. The control system 104 may include, for example, a processor 106, a pulse generator 108, and a drive unit 110. The pulse generator 108 forms electric pulses that may be input to one or more transducers (not shown) disposed in the catheter 102.

With some embodiments, mechanical energy from the drive unit 110 can be used to drive an imaging core (also not shown) disposed in the catheter 102. In at least some embodiments, electric signals transmitted from the one or more transducers may be input to the processor 106 for processing. In at least some embodiments, the processed electric signals from the one or more transducers can be used to form a series of images, described in more detail below. For example, a scan converter can be used to map scan line samples (e.g., radial scan line samples, or the like) to a two-dimensional Cartesian grid, which can be used as the basis for a series of IVUS images that can be displayed for a user.

In at least some embodiments, the processor 106 may also be used to control the functioning of one or more of the other components of the control system 104. For example, the processor 106 may be used to control at least one of the frequency or duration of the electrical pulses transmitted from the pulse generator 108, the rotation rate of the imaging core by the drive unit 110. Additionally, where IVUS imaging system 100 is configured for automatic pullback, the drive unit 110 can control the velocity and/or length of the pullback.

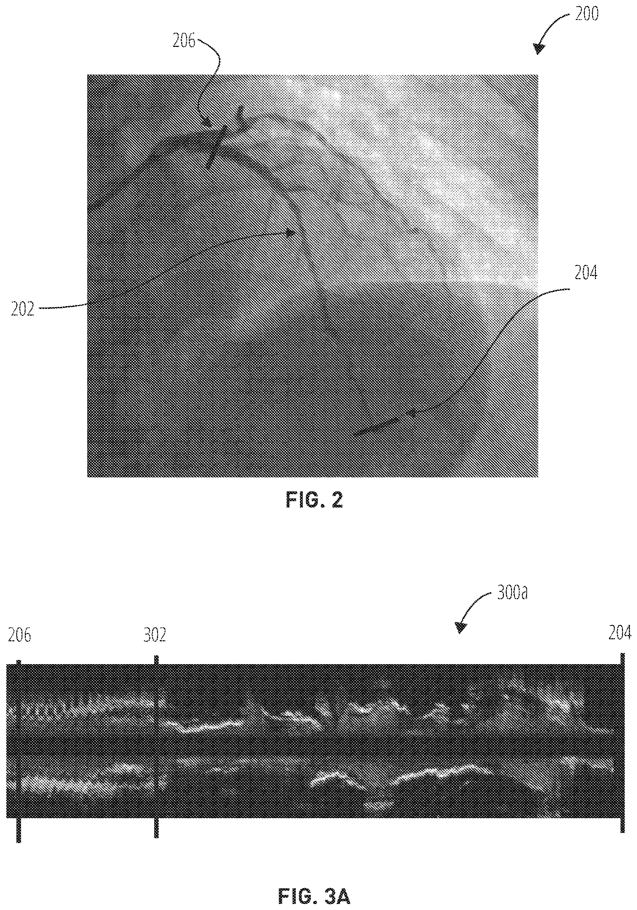

FIG. 2 illustrates an image 200 of a vessel 202 of a patient. As described, IVUS imaging systems (e.g., IVUS imaging system 100, or the like) are used to capture a series of images or a "recording" or a vessel, such as, vessel 202. For example, an IVUS catheter (e.g., catheter 102) is inserted into vessel 202 and a recording, or a series of IVUS images, is captured as the catheter 102 is pulled back from a distal end 204 to a proximal end 206. The catheter 102 can be pulled back manually or automatically (e.g., under control of drive unit 110, or the like).

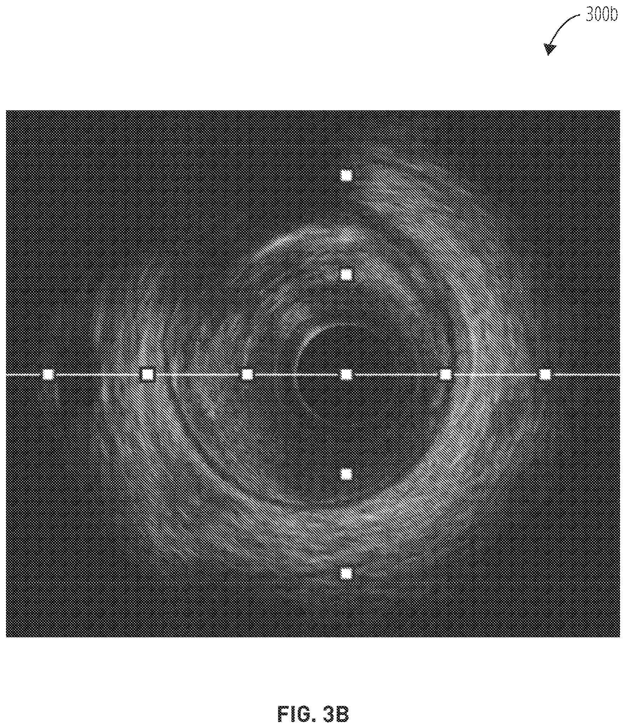

FIG. 3A and FIG. 3B illustrates two-dimensional (2D) representations of IVUS images of vessel 202. For example, FIG. 3A illustrates IVUS images 300a depicting a longitudinal view of the IVUS recording of vessel 202 between proximal end 206 and distal end 204.

FIG. 3B illustrates an image frame 300b depicting an on-axis (or short axis, or cross-section) view of vessel 202 at point 302. Said differently, image frame 300b is a single frame or single image from a series of IVUS images that can be captured between distal end 204 and proximal end 206 as described herein. As introduced above, the present disclosure provides systems and techniques to process raw IVUS images to identify regions of interest, such as, for example starting and ending points between which include frames of interest in a series of IVUS images.

For example, IVUS images 300a depicts an entire series of IVUS images taken of vessel 202 between distal end 204 and proximal end 206. IVUS images may be captured at several stages of a percutaneous coronary intervention (PCI). That is, IVUS may be employed pre-PCI, peri-PCI, or post-PCI. For example, IVUS may be employed to capture images of the state of the vessel 202 before a stent is implanted. In such an example, automatic assessments of the images can be performed (e.g., vessel border detection, lumen border detection, plaque burden detection, key frame identification, stent size and landing zone recommendations, stent expansion estimation, calcium detection, or the like). It is to be appreciated that this is a significant amount of information to convey to a user. Further, conveying this information along with the IVUS images (e.g., IVUS images 300a and 300b) in a manner that allows the user to visualize the vessel structure along with obstructions or stenosis is not provided in the prior art. Thus, the present disclosure provides an advantage in that the improved GUI provides for greater understanding of the vessel physiology and allows manipulation of the features of the image.

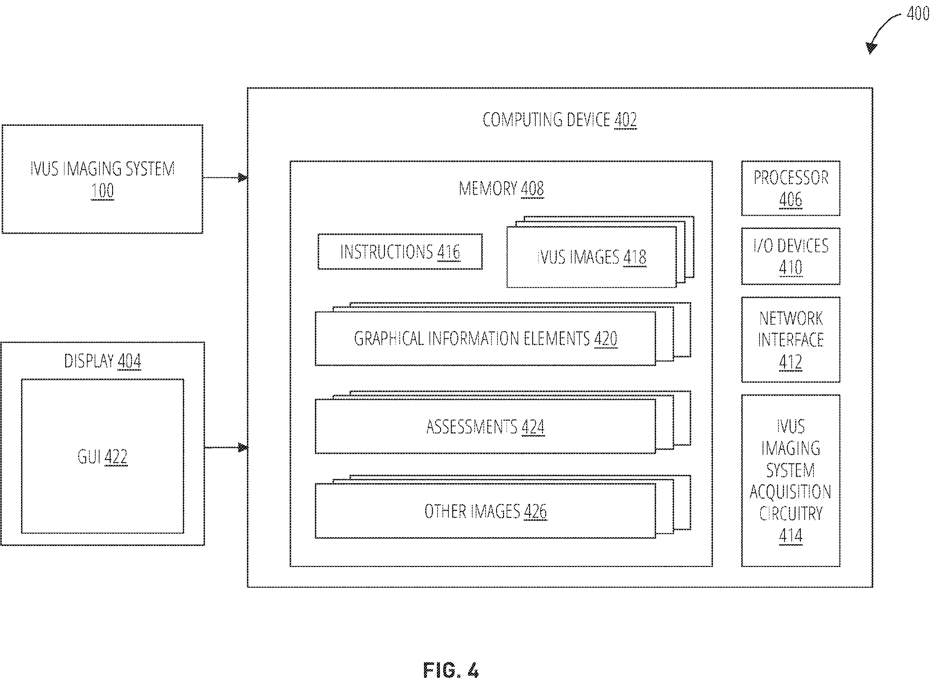

FIG. 4 illustrates an IVUS image visualization system 400, according to some embodiments of the present disclosure. In general, IVUS image visualization system 400 is a system for processing, annotating, and presenting IVUS images. IVUS image visualization system 400 can be implemented in a commercial IVUS guidance or navigation system, such as, for example, the AVVIGO ^{®} Guidance System available from Boston Scientific ^{®}. The present disclosure provides advantages over prior or conventional IVUS navigation systems in that the improved GUI will reduce the time needed for patients to be in treatment. For example, the present disclosure can be implemented in an IVUS navigation system to efficiently communicate information about vessel physiology to a user and allow the user to manipulate the information, thereby reducing the treatment time for the patient.

With some embodiments, IVUS image visualization system 400 could be implemented as part of control system 104. Alternatively, control system 104 could be implemented as part of IVUS image visualization system 400. As depicted, IVUS image visualization system 400 includes a computing device 402. Optionally, IVUS image visualization system 400 includes IVUS imaging system 100 and display 404.

Computing device 402 can be any of a variety of computing devices. In some embodiments, computing device 402 can be incorporated into and/or implemented by a console of display 404 (e.g., as a tablet computer, or the like). With some embodiments, computing device 402 can be a workstation or server communicatively coupled to IVUS imaging system 100 and/or display 404. With still other embodiments, computing device 402 can be provided by a cloud based computing device, such as, by a computing as a service system accessibly over a network (e.g., the Internet, an intranet, a wide area network, or the like). Computing device 402 can include processor 406, memory 408, input and/or output (I/O) devices 410, network interface 412, and IVUS imaging system acquisition circuitry 414.

The processor 406 may include circuity or processor logic, such as, for example, any of a variety of commercial processors. In some examples, processor 406 may include multiple processors, a multi-threaded processor, a multi-core processor (whether the multiple cores coexist on the same or separate dies), and/or a multiprocessor architecture of some other variety by which multiple physically separate processors are in some way linked. Additionally, in some examples, the processor 406 may include graphics processing portions and may include dedicated memory, multiple-threaded processing and/or some other parallel processing capability. In some examples, the processor 406 may be an application specific integrated circuit (ASIC) or a field programmable integrated circuit (FPGA).

The memory 408 may include logic, a portion of which includes arrays of integrated circuits, forming non-volatile memory to persistently store data or a combination of non-volatile memory and volatile memory. It is to be appreciated, that the memory 408 may be based on any of a variety of technologies. In particular, the arrays of integrated circuits included in memory 120 may be arranged to form one or more types of memory, such as, for example, dynamic random access memory (DRAM), NAND memory, NOR memory, or the like.

I/O devices 410 can be any of a variety of devices to receive input and/or provide output. For example, I/O devices 410 can include, a keyboard, a mouse, a joystick, a foot pedal, a display, a touch enabled display, a haptic feedback device, an LED, or the like.

Network interface 412 can include logic and/or features to support a communication interface. For example, network interface 412 may include one or more interfaces that operate according to various communication protocols or standards to communicate over direct or network communication links. Direct communications may occur via use of communication protocols or standards described in one or more industry standards (including progenies and variants). For example, network interface 412 may facilitate communication over a bus, such as, for example, peripheral component interconnect express (PCIe), non-volatile memory express (NVMe), universal serial bus (USB), system management bus (SMBus), SAS (e.g., serial attached small computer system interface (SCSI)) interfaces, serial AT attachment (SATA) interfaces, or the like. Additionally, network interface 412 can include logic and/or features to enable communication over a variety of wired or wireless network standards (e.g., 802.11 communication standards). For example, network interface 412 may be arranged to support wired communication protocols or standards, such as, Ethernet, or the like. As another example, network interface 412 may be arranged to support wireless communication protocols or standards, such as, for example, Wi-Fi, Bluetooth, ZigBee, LTE, 5G, or the like.

The IVUS imaging system acquisition circuitry 414 may include circuity including custom manufactured or specially programmed circuitry configured to receive or receive and send signals between IVUS imaging system 100 including indications of an IVUS run, a series of IVUS images, or a frame or frames of IVUS images.

Memory 408 can include instructions 416. During operation processor 406 can execute instructions 416 to cause computing device 402 to receive (e.g., from IVUS imaging system 100, or the like) a recording of an "IVUS run" and store the recording as IVUS images 418 in memory 408. For example, processor 406 can execute instructions 416 to receive information elements from IVUS imaging system 100 comprising indications of IVUS images captured by catheter 102 while being pulled back from distal end 204 to proximal end 206, which images comprising indications of the anatomy and/or structure of vessel 202 including vessel walls and plaque. It is to be appreciated that IVUS images 418 can be stored in a variety of image formats or even non-image formats or data structures that comprise indications of vessel 202. Further, IVUS images 418 includes several "frames" or individual images that, when represented co-linearly can be used to form an image of the vessel 202, such as, for example, as represented by IVUS images 300a and/or 300b.

The present disclosure provides to generate graphical information elements 420 from IVUS images 418 and to generate a GUI 422 to be displayed on display 404 based on the graphical information elements 420. Processor 406 can further be configured to execute instructions 416 to generate assessments 424 based on IVUS images 418. This will be described in greater detail below. However, in general, the assessments can include vessel boundary detection, lumen boundary detection, plaque burden determination, key frame identification, distances between key frames, stent detection, stent expansion estimates, calcium detection, among other assessments. As such, in some embodiments, graphical information elements 420 can be generated based on IVUS images 418 and assessments 424.

Memory 408 can also include other images 426, such as, for example, Angio images (e.g., image 200, or the like). With some examples, processor 406 can execute instructions 416 to generate a graphical information elements 420 comprising an indication of other images 426 and assessments 424. With some examples, the other images 426 can be linked or mapped to IVUS images 418 and the graphical information elements 420 can be overlaid over representations of frames of IVUS images 418 or other images 426.

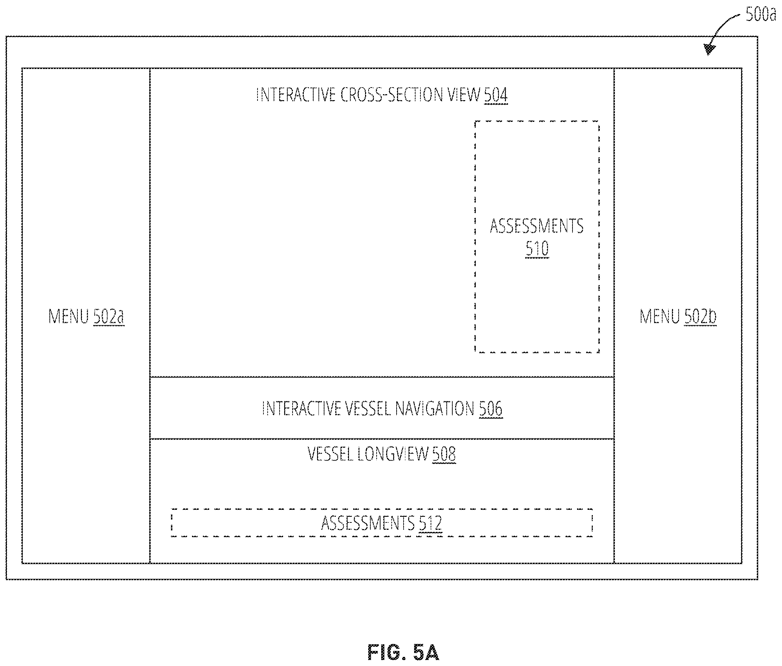

FIG. 5A illustrates a GUI 500a, which can be generated according to some embodiments of the present disclosure. For example, GUI 500a can be generated by IVUS image visualization system 400 as GUI 422 and displayed on display 404. As depicted, GUI 500a includes several graphical information elements 420, such as, menus 502a and 502b, interactive cross-section view 504, and interactive vessel navigation 506, and vessel long view 508.

With some embodiments, processor 406 can be configured to execute instructions 416 to generate GUI 500a. As another example, processor 406 can execute instructions 416 to generate GUI 500a responsive to an automatic plaque burden detection process. For example, with some embodiments, IVUS image visualization system 400 can be arranged to automatically detect the plaque burden of the vessel 202 represented by IVUS images 418 calcium (e.g., via machine learning, image classification, or the like).

Menu 502a can comprise GUI inputs such as button, drop down menus, selection icons, or the like. Menu 502a can include GUI input options to select measurement and annotation tools, length tools, modification reset buttons, or the like. Menu 502b can comprise GUI inputs such as buttons, drop down menus, selection icons, or the like. Menu 502b can include GUI inputs options to select views related to views of the IVUS images, layout options, annotations, navigation, dynamic review options, status of the computing device, or the like.

Interactive cross-section view 504 can comprise a cross-sectional view of a one (e.g., a frame, or the like) of IVUS images 418. For example, interactive cross-section view 504 can include image frame 300b and assessments 510 as well as indications of vessel and lumen borders. A detailed description of interactive vessel navigation 506 is provided below. However, in general, interactive vessel navigation 506 can include a navigation slider to navigation through IVUS images 418, which is linked to the interactive cross-section view 504. That is, as the slider is moved the image displayed in interactive cross-section view 504 changes to match the location indicated by the slider.

Vessel Longview 508 can comprise a longitudinal view of the vessel (e.g., vessel 202) represented by the IVUS images 418. For example, vessel long view 508 can include representations of a vessel and lumen profile as well as assessments 512. Vessel Longview 508 can include a graphical representation of IVUS images 300a. With some embodiments, the assessments 512 can be included to represent the detected plaque burden along the vessel 202. Additionally, vessel long view 508 can include key frame markers (e.g., locations of distal and proximal frames, such as, distal and proximal key frames, linearly along the series of IVUS images 418 can be indicated (e.g., with brackets, or the like).

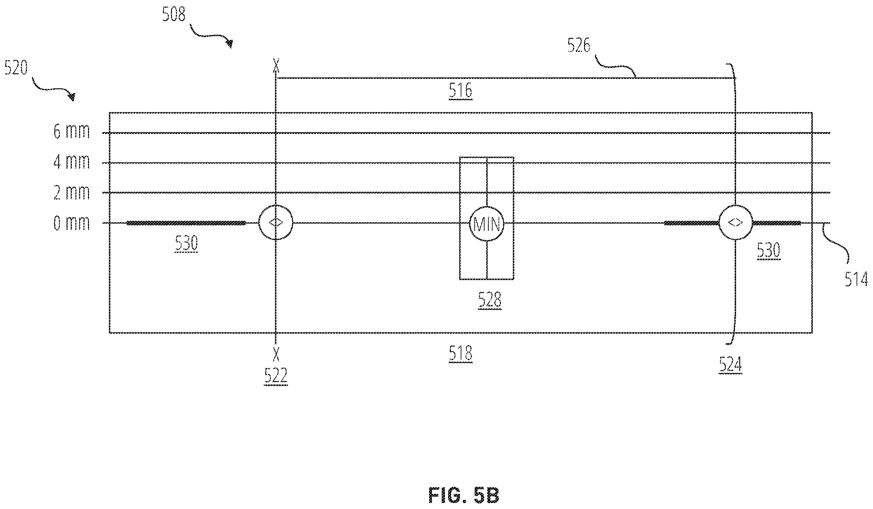

FIG. 5B illustrates an example vessel Longview 508, according to some embodiments of the present disclosure. IVUS image visualization system 400 can be configured to generate 508 as a graphical component of GUI 500a. As depicted, vessel long view 508 includes several GUI components (or sub-graphical components) including a central axis 514 about which longitudinal border profile 516 and longitudinal border profile mirror reflection 518 are disposed. Examples of longitudinal border profile 516 and 518 are given below. However, in general, 516 depicts or is representative of the detected borders (e.g., borders border 604, or the like) for the IVUS images 418. Longitudinal border profile mirror reflection 518 is a mirror reflection of the longitudinal border profile 516, thereby providing a more complete visualization of the vessel and lumen profile.

Vessel Longview 508 further includes scale 520 depicting the radius of the detected borders represented in longitudinal border profile 516. Additionally, vessel long view 508 includes proximal bracket end 522, distal bracket end 524, and minimum region 528. Each of the brackets are movable via user input (e.g., via I/O devices 410). With some examples, proximal bracket end 522 and distal bracket end 524 are automatically placed at an initial location based on their locations with respect to detected lumen size, detected stent in the vessel 202, or the like. Further, in some embodiments, proximal bracket end 522 and/or distal bracket end 524 can have one graphical representation when located in an area of vessel 202 with a detected plaque burden less than a threshold amount (e.g., 50%, or the like) and another, different, graphical representation when located in an area of vessel 202 with a detected plaque burden greater than or equal to the threshold amount. For example, proximal bracket end 522 is depicted having a different graphical representation than distal bracket end 524. As noted above, the proximal bracket end 522 and distal bracket end 524 are interactive in that their locations can be manipulated by a user (e.g., via input and/or output (I/O) devices 410, or the like). As such, processor 406 can execute instructions 416 to dynamically generate proximal bracket end 522 and/or distal bracket end 524 based on their manipulated locations with respect to the detected plaque burden and a threshold. For example, if a user moved proximal bracket end 522 more proximal such that proximal bracket end 522 was in the regions designated by plaque burden indicator 530 (e.g., less than a threshold amount of detected plaque burden) then processor 406 could execute instructions 416 to generate proximal bracket end 522 having a graphical representation like distal bracket end 524. Likewise, if a user moved distal bracket end 524 more proximal such that distal bracket end 524 was located outside the regions designated by plaque burden indicator 530 (e.g., greater than or equal to a threshold amount of detected plaque burden) then processor 406 could execute instructions 416 to generate proximal distal bracket end 524 having a graphical representation like distal proximal bracket end 522.

Lastly, vessel Longview 508 includes a graphical indication of the detected plaque burden, shown as plaque burden indicator 530. Furthermore, with some examples, ruler 526 can be enabled (e.g., by default, by input button selection, or the like) and can measure a distance between brackets (e.g., proximal bracket end 522 and distal bracket end 524).

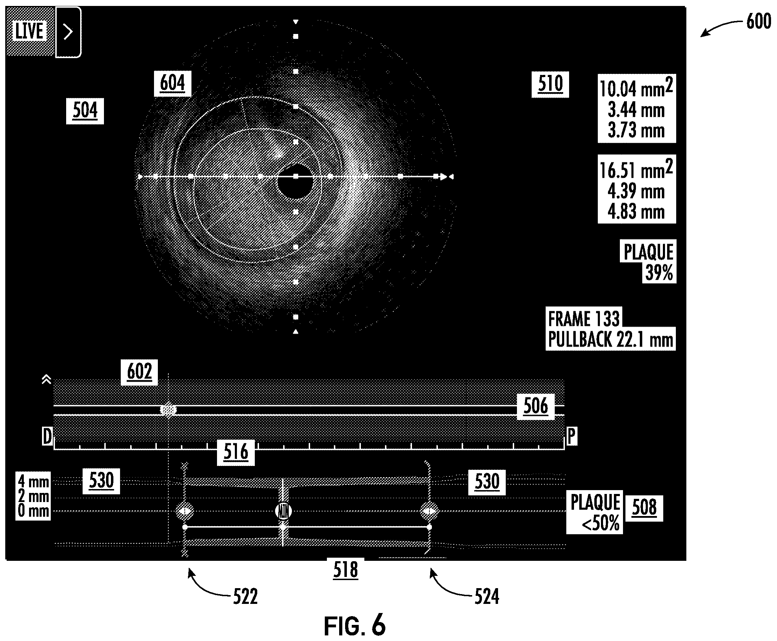

FIG. 6 illustrates a GUI 600, which can be generated according to some embodiments of the present disclosure. For example, GUI 600 can be generated by IVUS image visualization system 400 as GUI 422 and displayed on display 404. With some embodiments, responsive to detection of a plaque burden of the vessel 202, processor 406 can execute instructions 416 to generate graphical information elements 420 and GUI 600 from graphical information elements 420.

As depicted, GUI 600 interactive cross-section view 504 and interactive vessel navigation 506. Interactive cross-section view 504 includes a depiction of a cross-section view of a frame of the IVUS images 418 (e.g., corresponding to the location of slider 602) as well as various assessments 510 such as borders border 604 (e.g., vessel border, lumen border, etc.), plaque burden, frame number, pullback distance, or the like. Further, GUI 600 includes interactive vessel navigation 506, which includes an interactive slider 602 that can be used to navigate through the IVUS images 418 represented in GUI 600.

Additionally, GUI 600 includes vessel long view 508 depicting longitudinal border profile 516 and longitudinal border profile mirror reflection 518 as well as proximal bracket end 522 and distal bracket end 524 and plaque burden indicator 530. As can be seen, the plaque burden indicator 530 indicates regions of vessel 202 represented by IVUS images 418 where the detected plaque burden is less than a threshold amount (e.g., 50%). Further, the graphical representation of proximal bracket end 522 and distal bracket end 524 can depend upon their locations with respect to the detected plaque burden amount and the threshold level. In particular, as depicted in GUI 600, proximal bracket end 522 is located within an area of vessel 202 where the detected plaque burden is greater than or equal to the threshold level, and as such, has a first graphical representation (e.g., straight line with "X" at ends) while distal bracket end 524 is located within an area of vessel 202 where the detected plaque burden is less than the threshold level, and as such, has a second graphical representation (e.g., straight line with curved ends).

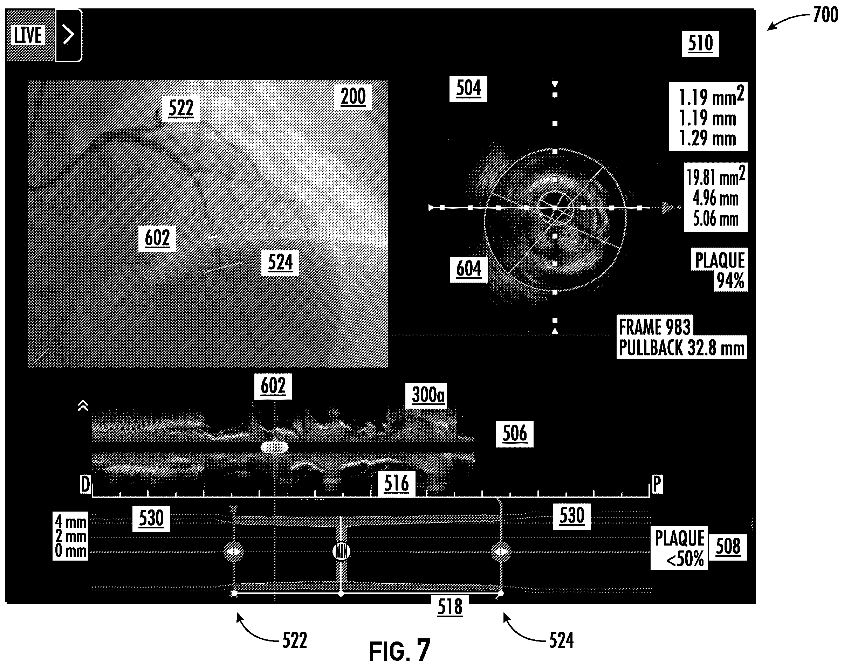

FIG. 7 illustrates a GUI 700, which can be generated according to some embodiments of the present disclosure. For example, GUI 700 can be generated by IVUS image visualization system 400 as GUI 422 and displayed on display 404. With some embodiments, responsive to detection of a plaque burden of the vessel 202, processor 406 can execute instructions 416 to generate graphical information elements 420 and GUI 700 from graphical information elements 420.

GUI 700 includes graphical elements like 600 in addition to an indication of an angiographic image of the vessel 202 represented by IVUS images 418. GUI 700 includes image 200 along with interactive cross-section view 504, interactive vessel navigation 506, and vessel Longview 508. With some examples, graphical indications of the distal and proximal key frames (e.g., proximal bracket end 522 and distal bracket end 524) can be represented on image 200. Further, as depicted, interactive vessel navigation 506 includes IVUS images 300a (e.g., the actual IVUS images 418).

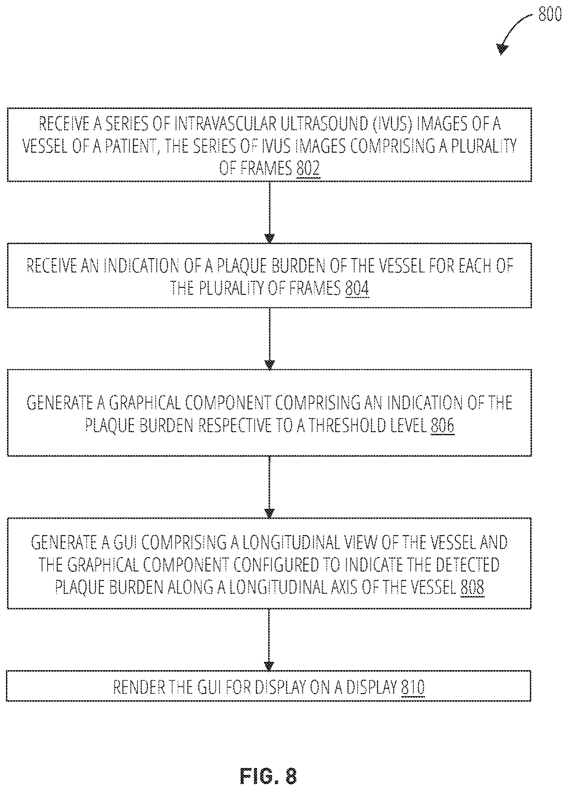

FIG. 8 illustrates a logic flow 800 to generate a GUI, according to some embodiments of the present disclosure. The logic flow 800 can be implemented by IVUS image visualization system 400 and will be described with reference to IVUS image visualization system 400 for clarity of presentation. However, it is noted that logic flow 800 could also be implemented by an IVUS guidance system different than IVUS image visualization system 400.

Logic flow 800 can begin at block 802. At block 802 "receive a series of intravascular ultrasound (IVUS) images of a vessel of a patient, the series of IVUS images comprising a plurality of frames" a series of IVUS images captured via an IVUS catheter percutaneously inserted in a vessel of a patent can be received. For example, information elements comprising indications of IVUS images 418 can be received from IVUS imaging system 100 where catheter 102 is (or was) percutaneously inserted into vessel 202. The IVUS images 418 can comprise frames of images representative of images captured while the catheter 102 is pulled back from distal end 204 to proximal end 206. Processor 406 can execute instructions 416 to receive information elements comprising indications of IVUS images 418 from IVUS imaging system 100, or directly from catheter 102 as may be the case.

Continuing to block 804 "receive an indication of a plaque burden of the vessel for each of the plurality of frames" an indication of a plaque burden of the vessel for each of the plurality of frames is received. For example, processor 406 can execute instructions 416 to receive (e.g., from a machine learning model, or the like) an indication of the plaque burden of the vessel based on the frames of IVUS images 418. Continuing to block 806 "generate a graphical component comprising an indication of the plaque burden respective to a threshold level" a graphical component comprising an indication of the plaque burden respective to a threshold level is generated. For example, processor 406 can execute instructions 416 to generate plaque burden indicator 530 comprising an indication of area of vessel 202 where the detected plaque burden is less than a threshold level. As a specific example, processor 406 can execute instructions 416 to generate plaque burden indicator 530 comprising a line overlaying representations of frames of IVUS images 418 where the detected plaque burden is less than a threshold level (e.g., 50%, or the like).

Continuing to block 808 "generate a GUI comprising a longitudinal view of the vessel and the graphical component configured to indicate the detected plaque burden along a longitudinal axis of the vessel" a GUI comprising a longitudinal view of the vessel and the graphical component configured to indicate the detected plaque burden along a longitudinal axis of the vessel is generated. For example, processor 406 can execute instructions 416 to generate GUI 422 comprising vessel long view 508 and indications (e.g., proximal bracket end 522, distal bracket end 524, plaque burden indicator 530, etc.) Continuing to block 810 "render the GUI for display on a display" the GUI can be rendered for display. For example, processor 406 can execute instructions 416 to generate GUI 422 for display by display 404.

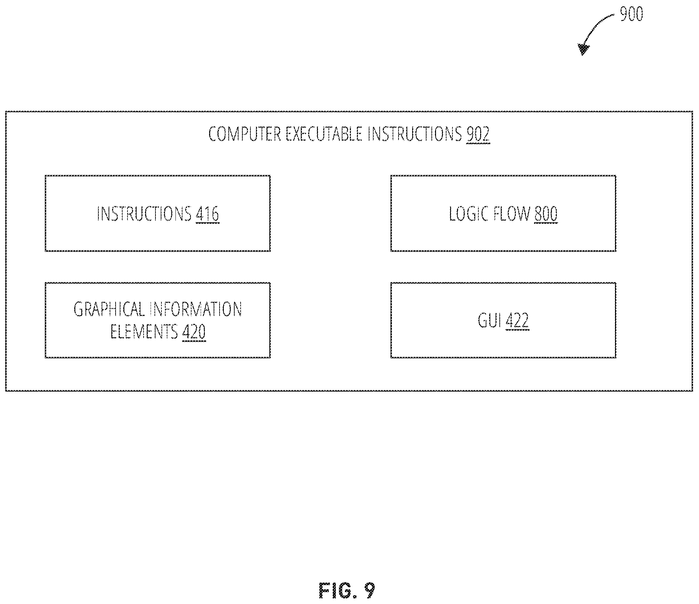

FIG. 9 illustrates computer-readable storage medium 900. Computer-readable storage medium 900 may comprise any non-transitory computer-readable storage medium or machine-readable storage medium, such as an optical, magnetic or semiconductor storage medium. In various embodiments, computer-readable storage medium 900 may comprise an article of manufacture. In some embodiments, computer-readable storage medium 900 may store computer executable instructions 902 with which circuitry (e.g., processor 106, processor 406, IVUS imaging system acquisition circuitry 414, and the like) can execute. For example, computer executable instructions 902 can include instructions to implement operations described with respect to instructions 416, logic flow 800, graphical information elements 420, and/or GUI 422. Examples of computer-readable storage medium 900 or machine-readable storage medium may include any tangible media capable of storing electronic data, including volatile memory or non-volatile memory, removable or non-removable memory, erasable or non-erasable memory, writeable or re-writeable memory, and so forth. Examples of computer executable instructions 902 may include any suitable type of code, such as source code, compiled code, interpreted code, executable code, static code, dynamic code, object-oriented code, visual code, and the like.

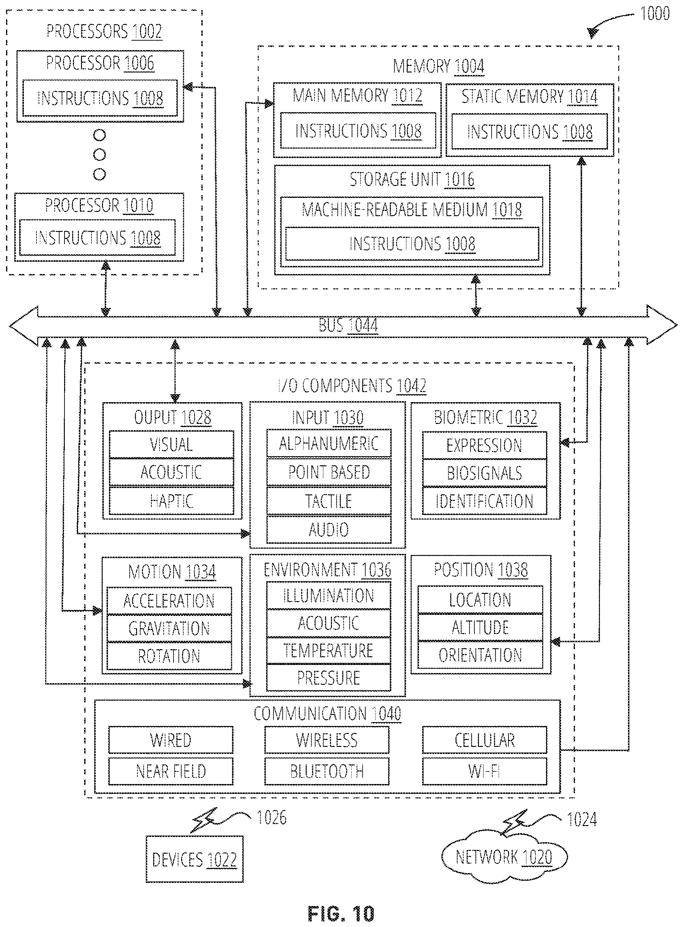

FIG. 10 illustrates a diagrammatic representation of a machine 1000 in the form of a computer system within which a set of instructions may be executed for causing the machine to perform any one or more of the methodologies discussed herein. More specifically, FIG. 10 shows a diagrammatic representation of the machine 1000 in the example form of a computer system, within which instructions 1008 (e.g., software, a program, an application, an applet, an app, or other executable code) for causing the machine 1000 to perform any one or more of the methodologies discussed herein may be executed. For example, the instructions 1008 may cause the machine 1000 to execute logic flow 800 of FIG. 8, instructions 416 of FIG. 4. More generally, the instructions 1008 may cause the machine 1000 to generate GUIs with functionality and behavior as described herein during a pre-PCI, peri-PCI, or post-PCI using IVUS. It is noted that the present disclosure provides specific and discrete implementations of GUI representations and behavior that is a significant improvement over the prior art. In particular, the present disclosure provides an improvement to computing technology in that GUIs provide greater visibility and navigation of IVUS images.

The instructions 1008 transform the general, non-programmed machine 1000 into a particular machine 1000 programmed to carry out the described and illustrated functions in a specific manner. In alternative embodiments, the machine 1000 operates as a standalone device or may be coupled (e.g., networked) to other machines. In a networked deployment, the machine 1000 may operate in the capacity of a server machine or a client machine in a server-client network environment, or as a peer machine in a peer-to-peer (or distributed) network environment. The machine 1000 may comprise, but not be limited to, a server computer, a client computer, a personal computer (PC), a tablet computer, a laptop computer, a netbook, a set-top box (STB), a PDA, an entertainment media system, a cellular telephone, a smart phone, a mobile device, a wearable device (e.g., a smart watch), a smart home device (e.g., a smart appliance), other smart devices, a web appliance, a network router, a network switch, a network bridge, or any machine capable of executing the instructions 1008, sequentially or otherwise, that specify actions to be taken by the machine 1000. Further, while only a single machine 1000 is illustrated, the term "machine" shall also be taken to include a collection of machines 1000 that individually or jointly execute the instructions 1008 to perform any one or more of the methodologies discussed herein.

The machine 1000 may include processors 1002, memory 1004, and I/O components 1042, which may be configured to communicate with each other such as via a bus 1044. In an example embodiment, the processors 1002 (e.g., a Central Processing Unit (CPU), a Reduced Instruction Set Computing (RISC) processor, a Complex Instruction Set Computing (CISC) processor, a Graphics Processing Unit (GPU), a Digital Signal Processor (DSP), an ASIC, a Radio-Frequency Integrated Circuit (RFIC), another processor, or any suitable combination thereof) may include, for example, a processor 1006 and a processor 1010 that may execute the instructions 1008. The term "processor" is intended to include multi-core processors that may comprise two or more independent processors (sometimes referred to as "cores") that may execute instructions contemporaneously. Although FIG. 10 shows multiple processors 1002, the machine 1000 may include a single processor with a single core, a single processor with multiple cores (e.g., a multi-core processor), multiple processors with a single core, multiple processors with multiples cores, or any combination thereof.

The memory 1004 may include a main memory 1012, a static memory 1014, and a storage unit 1016, both accessible to the processors 1002 such as via the bus 1044. The main memory 1004, the static memory 1014, and storage unit 1016 store the instructions 1008 embodying any one or more of the methodologies or functions described herein. The instructions 1008 may also reside, completely or partially, within the main memory 1012, within the static memory 1014, within machine-readable medium 1018 within the storage unit 1016, within at least one of the processors 1002 (e.g., within the processor's cache memory), or any suitable combination thereof, during execution thereof by the machine 1000.

The I/O components 1042 may include a wide variety of components to receive input, provide output, produce output, transmit information, exchange information, capture measurements, and so on. The specific I/O components 1042 that are included in a particular machine will depend on the type of machine. For example, portable machines such as mobile phones will likely include a touch input device or other such input mechanisms, while a headless server machine will likely not include such a touch input device. It will be appreciated that the I/O components 1042 may include many other components that are not shown in FIG. 10. The I/O components 1042 are grouped according to functionality merely for simplifying the following discussion and the grouping is in no way limiting. In various example embodiments, the I/O components 1042 may include output components 1028 and input components 1030. The output components 1028 may include visual components (e.g., a display such as a plasma display panel (PDP), a light emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components 1030 may include alphanumeric input components (e.g., a keyboard, a touch screen configured to receive alphanumeric input, a photo-optical keyboard, or other alphanumeric input components), point-based input components (e.g., a mouse, a touchpad, a trackball, a joystick, a motion sensor, or another pointing instrument), tactile input components (e.g., a physical button, a touch screen that provides location and/or force of touches or touch gestures, or other tactile input components), audio input components (e.g., a microphone), and the like.

In further example embodiments, the I/O components 1042 may include biometric components 1032, motion components 1034, environmental components 1036, or position components 1038, among a wide array of other components. For example, the biometric components 1032 may include components to detect expressions (e.g., hand expressions, facial expressions, vocal expressions, body gestures, or eye tracking), measure biosignals (e.g., blood pressure, heart rate, body temperature, perspiration, or brain waves), identify a person (e.g., voice identification, retinal identification, facial identification, fingerprint identification, or electroencephalogram-based identification), and the like. The motion components 1034 may include acceleration sensor components (e.g., accelerometer), gravitation sensor components, rotation sensor components (e.g., gyroscope), and so forth. The environmental components 1036 may include, for example, illumination sensor components (e.g., photometer), temperature sensor components (e.g., one or more thermometers that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment. The position components 1038 may include location sensor components (e.g., a GPS receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 1042 may include communication components 1040 operable to couple the machine 1000 to a network 1020 or devices 1022 via a coupling 1024 and a coupling 1026, respectively. For example, the communication components 1040 may include a network interface component or another suitable device to interface with the network 1020. In further examples, the communication components 1040 may include wired communication components, wireless communication components, cellular communication components, Near Field Communication (NFC) components, Bluetooth^{®} components (e.g., Bluetooth^{®} Low Energy), Wi-Fi^{®} components, and other communication components to provide communication via other modalities. The devices 1022 may be another machine or any of a wide variety of peripheral devices (e.g., a peripheral device coupled via a USB).

Moreover, the communication components 1040 may detect identifiers or include components operable to detect identifiers. For example, the communication components 1040 may include Radio Frequency Identification (RFID) tag reader components, NFC smart tag detection components, optical reader components (e.g., an optical sensor to detect one-dimensional bar codes such as Universal Product Code (UPC) bar code, multi-dimensional bar codes such as Quick Response (QR) code, Aztec code, Data Matrix, Dataglyph, MaxiCode, PDF417, Ultra Code, UCC RSS-2D bar code, and other optical codes), or acoustic detection components (e.g., microphones to identify tagged audio signals). In addition, a variety of information may be derived via the communication components 1040, such as location via Internet Protocol (IP) geolocation, location via Wi-Fi^{®} signal triangulation, location via detecting an NFC beacon signal that may indicate a particular location, and so forth.

The various memories (i.e., memory 1004, main memory 1012, static memory 1014, and/or memory of the processors 1002) and/or storage unit 1016 may store one or more sets of instructions and data structures (e.g., software) embodying or utilized by any one or more of the methodologies or functions described herein. These instructions (e.g., the instructions 1008), when executed by processors 1002, cause various operations to implement the disclosed embodiments.

As used herein, the terms "machine-storage medium," "device-storage medium," "computer-storage medium" mean the same thing and may be used interchangeably in this disclosure. The terms refer to a single or multiple storage devices and/or media (e.g., a centralized or distributed database, and/or associated caches and servers) that store executable instructions and/or data. The terms shall accordingly be taken to include, but not be limited to, solid-state memories, and optical and magnetic media, including memory internal or external to processors. Specific examples of machine-storage media, computer-storage media and/or device-storage media include non-volatile memory, including by way of example semiconductor memory devices, e.g., erasable programmable read-only memory (EPROM), electrically erasable programmable read-only memory (EEPROM), FPGA, and flash memory devices; magnetic disks such as internal hard disks and removable disks; magnetooptical disks; and CD-ROM and DVD-ROM disks. The terms "machine-storage media," "computer-storage media," and "device-storage media" specifically exclude carrier waves, modulated data signals, and other such media, at least some of which are covered under the term "signal medium" discussed below.

In various example embodiments, one or more portions of the network 1020 may be an ad hoc network, an intranet, an extranet, a VPN, a LAN, a WLAN, a WAN, a WWAN, a MAN, the Internet, a portion of the Internet, a portion of the PSTN, a plain old telephone service (POTS) network, a cellular telephone network, a wireless network, a Wi-Fi^{®} network, another type of network, or a combination of two or more such networks. For example, the network 1020 or a portion of the network 1020 may include a wireless or cellular network, and the coupling 1024 may be a Code Division Multiple Access (CDMA) connection, a Global System for Mobile communications (GSM) connection, or another type of cellular or wireless coupling. In this example, the coupling 1024 may implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High Speed Packet Access (HSPA), Worldwide Interoperability for Microwave Access (WiMAX), Long Term Evolution (LTE) standard, others defined by various standard-setting organizations, other long range protocols, or other data transfer technology.

The instructions 1008 may be transmitted or received over the network 1020 using a transmission medium via a network interface device (e.g., a network interface component included in the communication components 1040) and utilizing any one of several well-known transfer protocols (e.g., hypertext transfer protocol (HTTP)). Similarly, the instructions 1008 may be transmitted or received using a transmission medium via the coupling 1026 (e.g., a peer-to-peer coupling) to the devices 1022. The terms "transmission medium" and "signal medium" mean the same thing and may be used interchangeably in this disclosure. The terms "transmission medium" and "signal medium" shall be taken to include any intangible medium that can store, encoding, or carrying the instructions 1008 for execution by the machine 1000, and includes digital or analog communications signals or other intangible media to facilitate communication of such software. Hence, the terms "transmission medium" and "signal medium" shall be taken to include any form of modulated data signal, carrier wave, and so forth. The term "modulated data signal" means a signal that has one or more of its characteristics set or changed in such a matter as to encode information in the signal.

Terms used herein should be accorded their ordinary meaning in the relevant arts, or the meaning indicated by their use in context, but if an express definition is provided, that meaning controls.

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to one or multiple ones. Additionally, the words "herein," "above," "below" and words of similar import, when used in this application, refer to this application as a whole and not to any portions of this application. When the claims use the word "or" in reference to a list of two or more items, that word covers all the following interpretations of the word: any of the items in the list, all the items in the list and any combination of the items in the list, unless expressly limited to one or the other. Any terms not expressly defined herein have their conventional meaning as commonly understood by those having skill in the relevant art(s).

By using genuine models of anatomy more accurate surgical plans may be developed than through statistical modeling.

Terms used herein should be accorded their ordinary meaning in the relevant arts, or the meaning indicated by their use in context, but if an express definition is provided, that meaning controls.

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to one or multiple ones. Additionally, the words "herein," "above," "below" and words of similar import, when used in this application, refer to this application as a whole and not to any portions of this application. When the claims use the word "or" in reference to a list of two or more items, that word covers all the following interpretations of the word: any of the items in the list, all the items in the list and any combination of the items in the list, unless expressly limited to one or the other. Any terms not expressly defined herein have their conventional meaning as commonly understood by those having skill in the relevant art(s).

By using genuine models of anatomy more accurate surgical plans may be developed than through statistical modeling.

Terms used herein should be accorded their ordinary meaning in the relevant arts, or the meaning indicated by their use in context, but if an express definition is provided, that meaning controls.

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to one or multiple ones. Additionally, the words "herein," "above," "below" and words of similar import, when used in this application, refer to this application as a whole and not to any portions of this application. When the claims use the word "or" in reference to a list of two or more items, that word covers all the following interpretations of the word: any of the items in the list, all the items in the list and any combination of the items in the list, unless expressly limited to one or the other. Any terms not expressly defined herein have their conventional meaning as commonly understood by those having skill in the relevant art(s).

The following aspects are preferred embodiments of the present invention.
1. A method, comprising:
   receiving a series of intravascular ultrasound (IVUS) images of a vessel of a patient, the series of IVUS images comprising a plurality of frames;
   receiving, for each of the plurality of frames, an indication of plaque burden in the vessel;
   generating a graphical component comprising an indication of the plaque burden respective to a threshold amount;
   generating a GUI comprising a longitudinal view of the vessel and the graphical component configured to indicate the detected plaque burden along a longitudinal axis of the vessel; and
   rendering the GUI for display on a display.
2. The method of aspect 1, wherein the graphical component is a first graphical component, the method comprising:
   generating a second graphical component comprising the longitudinal view of the vessel; and
   generating the GUI comprising the first graphical component and the second graphical component.
3. The method of aspect 2, generating the GUI comprising overlaying the first graphical component over a portion of the second graphical component to indicate the plaque burden along the longitudinal axis of the vessel.
4. The method of any one of aspects 2 or 3, wherein the first graphical component comprises a line disposed along a portion of the longitudinal axis of the vessel.
5. The method of aspect 4, comprising:
   identifying, for each of the plurality of frames, whether the plaque burden is less than a threshold level; and
   overlaying the line over portions of the second graphical component associated with the ones of the plurality of frames where the plaque burden is less than the threshold level.
6. The method of aspect 5, wherein the second graphical component comprises an indication of a distal bracket, a proximal bracket, a minimum region, a vessel profile view and a mirror of the vessel profile view.
7. The method of aspect 6, wherein the vessel profile view comprises a graphical representation of a vessel border and a lumen border of the vessel along the longitudinal axis.
8. The method of any one of aspects 6 or 7, wherein the distal bracket comprises one of a plurality of bracket graphical representations selected based on the location of the distal bracket relative to the plaque burden and the threshold level.
9. The method of aspect 8, comprising:
   identifying a location of the distal bracket;
   determining whether the distal bracket is associated with a frame of the plurality of frames where the plaque burden is less than the threshold level; and
   selecting a first one of the plurality of bracket graphical representations based on a determination that the distal bracket is associated with a frame of the plurality of frames where the plaque burden is less than the threshold level; or
   selecting a second one of the plurality of bracket graphical representations based on a determination that the distal bracket is not associated with a frame of the plurality of frames where the plaque burden is less than the threshold level.
10. The method of any one of aspects 6 to 9, wherein the proximal bracket comprises one of a plurality of graphical representations selected based on the location of the proximal bracket relative to the plaque burden and the threshold level.
11. The method of aspect 10, comprising:
   identifying a location of the proximal bracket;
   determining whether the proximal bracket is associated with a frame of the plurality of frames where the plaque burden is less than the threshold level; and
   selecting a first one of the plurality of bracket graphical representations based on a determination that the proximal bracket is associated with a frame of the plurality of frames where the plaque burden is less than the threshold level; or
   selecting a second one of the plurality of bracket graphical representations based on a determination that the proximal bracket is not associated with a frame of the plurality of frames where the plaque burden is less than the threshold level.
12. The method of any one of aspects 1 to 11, comprising:
   generating a third graphical component comprising a cross-section view of a one of the plurality of frames; and
   generating the GUI comprising the first graphical component, the second graphical component, and the third graphical component.
13. The method of any one of aspects 1 to 12, wherein the GUI comprises an angiographic image of the vessel.
14. An apparatus, comprising a processor coupled to a memory, the memory comprising instructions executable by the processor, the processor configured to couple to an intravascular ultrasound (IVUS) imaging system and configured to execute the instructions, which instructions when executed cause the processor to implement the method of any one of aspects 1 to 13.
15. At least one machine readable storage device, comprising a plurality of instructions that in response to being executed by a processor of an intravascular ultrasound (IVUS) imaging system cause the processor to implement the method of any one of aspects 1 to 13.

## Claims

1. An apparatus, comprising a processor coupled to a memory, the memory comprising instructions executable by the processor, the processor configured to couple to an intravascular ultrasound, IVUS, imaging system and configured to execute the instructions, which instructions when executed cause the apparatus to:
receive a series of intravascular ultrasound, IVUS, images of a vessel of a patient, the series of IVUS images comprising a plurality of frames;
generate a GUI comprising a longitudinal view of the vessel; and
render the GUI for display on a display.

2. The apparatus of claim 1, wherein the longitudinal view of the vessel comprises a vessel profile view, the vessel profile view comprising a graphical representation of a vessel border and a lumen border of the vessel along a longitudinal axis of the vessel.

3. The apparatus of claim 1 or 2, wherein the longitudinal view of the vessel comprises a graphical indication of the detected plaque burden.

4. The apparatus of claim 3, wherein the graphical indication of the detected plaque burden comprises a line overlying representations of frames of the IVUS images where a detected plaque burden is less than a threshold level.

5. The apparatus of any of the previous claims, wherein the longitudinal view of the vessel comprises a scale depicting the radius of detected borders represented in a longitudinal border profile.

6. The apparatus of claim 5, wherein longitudinal view of the vessel comprises a proximal bracket end, a distal bracket end, and a minimum region, wherein each of the bracket ends are movable via user input.

7. The apparatus of claim 6, wherein the instructions when executed cause the apparatus to automatically place the proximal bracket end and the distal bracket end at initial positions based on their location with respect to detected lumen size and/or a detected stent in the vessel.

8. The apparatus of claim 6 or 7, wherein the proximal bracket end and/or the distal bracket end has one graphical representation when located in an area of a vessel with a detected plaque burden less than a threshold amount and another, different graphical representation when located in an area of the vessel with a detected plaque burden greater than or equal to the threshold amount.

9. The apparatus of any one of the preceding claims, wherein the instructions when executed cause the apparatus to:
generate a graphical component comprising a cross-section view of one of the plurality of frames,
wherein the GUI further comprises the graphical component.

10. The apparatus of claim 9, wherein the GUI comprises a navigation slider to navigate through the series of IVUS images which is linked to the cross-section view, so that the IVUS image displayed in the cross-section view changes to match the location indicated by the slider as the slider is moved.

11. The apparatus of claim 10, wherein the GUI comprises an interactive vessel navigation including the navigation slider, the interactive vessel navigation including IVUS images.

12. The apparatus of any one of claims 9 to 11, wherein the cross-section view comprises assessments, the assessments comprising one or more of vessel border, lumen border, plaque burden, frame number, and pullback distance.

13. The apparatus of any one of the preceding claims, wherein the longitudinal view of the vessel comprises one or more bracket, wherein the one or more bracket indicates one or more of a location of a distal frame, a location of a proximal frame, a location of a distal key frame, and a location of a proximal key frame.

14. Computer program comprising instructions which, when the program is executed by an apparatus comprising a processor, cause the apparatus to
receive a series of intravascular ultrasound, IVUS, images of a vessel of a patient, the series of IVUS images comprising a plurality of frames;
generate a GUI comprising a longitudinal view of the vessel; and
render the GUI for display on a display.

15. A computer readable storage medium comprising the computer program of claim 14.
